(19) Europäisches Patentamt / European Patent Office / Office européen des brevets

(11) **EP 2 189 789 A1**

(12) **DEMANDE DE BREVET EUROPEEN**

(43) Date de publication:
26.05.2010 Bulletin 2010/21

(51) Int Cl.:
*G01N 33/12* *(2006.01)*

(21) Numéro de dépôt: **09290878.9**

(22) Date de dépôt: **20.11.2009**

(84) Etats contractants désignés:
AT BE BG CH CY CZ DE DK EE ES FI FR GB GR
HR HU IE IS IT LI LT LU LV MC MK MT NL NO PL
PT RO SE SI SK SM TR
Etats d'extension désignés:
AL BA RS

(30) Priorité: **20.11.2008 FR 0806497**

(71) Demandeur: **Sedna**
**13382 Marseille Cedex 13 (FR)**

(72) Inventeurs:
• **Autheman, Clément**
**13382 Marseille Cédex 13 (FR)**
• **Widmann, Leslie**
**13382 Marseille Cédex 13 (FR)**

(74) Mandataire: **Demulsant, Xavier**
**Dejade & Biset**
**35, rue de Châteaudun**
**75009 Paris (FR)**

(54) **Procédé et dispositif de contrôle de la qualité de fraîcheur du poisson**

(57) Procédé de contrôle non destructif de la qualité du poisson, ce procédé comprenant au moins une mesure biométrique du poisson, **caractérisé en ce qu'**il comprend une étape de détection de l'oeil du poisson par un algorithme de détection d'arcs circulaires basés sur les contours, un cumul des centroïdes supposés pouvoir être le centre d'une pupille étant ensuite effectué, les zones de cumul important étant extraites, l'adresse de leur centroïdes étant moyennée pour obtenir le centre le plus probable.

# Fig.3

EP 2 189 789 A1

**Description**

L'invention se rapporte à un procédé et une installation de contrôle non destructif de la qualité de fraîcheur de poisson (parfois dénommée « qualité du poisson »).

Le poisson est un produit fragile dont la dégradation, très rapide, dépend de nombreux facteurs. Selon le type de capture, la pratique ou non de la saignée, le nettoyage ou le glaçage, le nombre de manipulations, la qualité du poisson débarqué ainsi que la rapidité de son vieillissement seront différentes.

L'altération du poisson met en oeuvre des phénomènes physiques, chimiques et bactériologiques. Elle se traduit par des changements d'aspect, l'apparition d'odeurs et de goûts anormaux. Les seuils d'apparition des odeurs sont très faibles: 600 ppb pour le triméthylamine par exemple.

L'évaluation de la fraîcheur du poisson est généralement sensorielle et subjective. Elle est effectuée par des experts, au sein même de la criée, la qualité d'un lot étant le critère prépondérant qui détermine sa mise à prix à la vente. L'expert humain effectue une analyse essentiellement visuelle, avec les risques de subjectivité et de manque de reproductibilité qui en découlent.

On connaît également, dans la littérature, plusieurs méthodes pour mesurer plus objectivement la qualité du poisson. Ces méthodes peuvent être regroupées en quatre grandes catégories : méthodes sensorielles, méthodes microbiologiques, méthodes mettant en oeuvre des mesures de paramètres biochimiques, méthodes mettant en oeuvre des mesures de paramètres physiques.

Méthodes sensorielles

La « fraîcheur » du poisson doit intuitivement avoir un lien avec les caractéristiques organoleptiques, c'est-à-dire l'ensemble des impressions sensorielles, visuelles, olfactives, et gustatives que procure le produit à sa dégustation.

Dans cet esprit, dans les années 80, l'ISPTM a proposé une description cotée des caractères d'altération du poisson, à partir d'observations à l'état cru et de l'évaluation de l'odeur et de la saveur à l'état cuit (Nicolle et al, Les conserves des produits de la mer, ISSN 0998-4089, disponible à l'adresse www.ifremer.fr). Un tableau de cotation analogue était publié en 1970, sur la base du règlement 2455/70 du conseil de l'Europe.

Les méthodes sensorielles sont les plus utilisées, dès les années 70 (schéma UE introduit par les décisions du conseil 103/76 et 104/76). Dans une première mise en pratique de ces méthodes sensorielles, pour la fraîcheur du poisson cru, un tableau de cotation européen (directive européenne 2406/96) permet de classer les produits en trois catégories : E (extra), A et B (article 4 de la directive 2406/96). Par exemple, pour les poissons blancs, les catégories de fraîcheur sont déterminées par examen de la pigmentation de la peau, par l'aspect du mucus cutané, la convexité de l'oeil, la transparence de la cornée, la couleur des branchies, l'odeur des branchies, l'élasticité de la chair. Ce règlement 2406/96 du conseil a été modifié par le règlement 323/97 du 21 février 1997 et par le règlement 2578/2000 du 17 novembre 2000. Une présentation de ces règlements du conseil de l'union européenne peut être trouvée à l'adresse www.ifremer.fr/bibliomer.

Dans une deuxième mise en oeuvre des méthodes sensorielles, dite méthode de l'indice de qualité (*Quality Index Méthod* QIM), les descriptions sont précises, subjectives, basées sur des paramètres sensoriels significatifs et déterminées pour des espèces en particulier. Cette méthode QIM a été développée au départ par l'unité de recherche alimentaire de Tasmanie en Australie. Pour une description de la procédure QIM, on peut se reporter aux normes ISO 5492 :1992 et 8586.1 et 8586.2 :2008 disponibles à l'adresse www.iso.org. La procédure QIM a été mise en oeuvre pour un grand nombre d'espèces de poissons. On peut se reporter, par exemple aux documents suivants : Sykes et al, "Assessment of European cuttlefish nutritional value and freshness under ice storage using a developed QIM and biochemical methods", LUVT Food Science and Technology, 2008 *;* Bonilla et al, "Development of QIM scheme for fresh cod fillets and application in shelf life study", Food Control 18, pp. 352-358, 2007*;* Pires et al, "Development of new QIM schemes for cuttlefish and broadtail short fin squid", Food control 17, pp. 942-949, 2006.

Certaines ventes électroniques à la criée en Europe appliquent la méthode d'indice de qualité.

L'avantage de la QIM est qu'une fois établie pour une espèce de poisson, elle peut être consultée par Internet et utilisée pour prédire le temps de stockage en glace.

L'inconvénient de la méthode de l'indice de qualité est de ne pas être adapté aux ventes à la criée et de ne couvrir qu'un nombre limité d'espèces. D'une manière générale, les méthodes sensorielles nécessitent un groupe de personnes expertes et entraînées, et sont longues et coûteuses.

Méthodes microbiologiques

Les méthodes microbiologiques visent à évaluer la présence possible de bactéries ou micro-organismes pouvant avoir des conséquences sur la santé publique. Les méthodes microbiologiques permettent notamment de déceler une éventuelle rupture de la chaîne du froid.

Les poissons pêchés dans des eaux non polluées ne portent que très rarement des bactéries pathogènes pour l'homme, à l'exception de *Vibrio parahaemolyticus*, ou de *Clostridium botulinum* présent notamment dans les boues marines. Les poissons pêchés dans les eaux polluées peuvent héberger sur la peau ou dans le tractus digestif de nombreux pathogènes humains (*E. Coli, Salomella, Shigella, Staphylococcus*), ainsi que de bactéries pathogènes originaires de l'eau : *Erysipleas, Leptospira, Pasteurella, Aeromonas, Pseudomonas, Mycobacetrium.*

Le respect des procédures doit cependant permettre de s'affranchir de ces bactéries et microorganismes, de sorte que les infections et intoxications bactériennes sont très rares. La mise en oeuvre internationale de la démarche HACCP permet d'identifier, d'évaluer et de contrôler les risques significatifs pour la sécurité sanitaire (www.ipfsaph.org).

Les examens bactériologiques les plus courants sont les suivants : flore totale ou flore mésophile aérobie totale (FMAT), détection de bactéries d'altération, présence de bactéries pathogènes.

Les examens bactériologiques sont complexes, longs, coûteux et requièrent des compétences pour leur exécution et l'interprétation des résultats. Les données microbiologiques ne fournissent pas en général d'informations sur l'appétence du poisson.

Méthodes mettant en oeuvre des mesures de paramètres biochimiques

Les méthodes mettant en oeuvre des mesures de paramètres biochimiques sont très variées : dosage de l'histamine, dosage des amines basiques totales (triméthylamine, diméthylamine, ammoniac), analyse des catabolites de nulécotides (inosine monophosphate, hypoxanthine), mesures de la rancidité oxydative (hydroperoxydes de lipides, aldéhydes, cétones, acides gras à chaîne courte). Des appareillages complexes sont nécessaires pour la mise en oeuvre de ces méthodes (par exemple spectroscopie visible ou proche infra rouge, chromatographie).

L'analyse des catabolites de nucléotides peut être effectuée à l'aide de biocapteurs, pour le calcul de l'indice $K_I$. Cet indicateur de fraîcheur tient compte du niveau de dégradation de l'ATP et des produits issus de l'action autolytique des enzymes endogènes. Un exemple de mise en oeuvre est décrit par Okuma et al, Flow system for fish freshness determination based on double multienzyme reactor electrodes, Biosensors & bioelectronics 17, pp. 367-372, 2002. Un exemple de procédé de dosage d'hypoxanthine est décrit dans le document WO 2008056990. Un exemple de procédé de mesure de l'indice K est décrit dans le document EP 137677.

La réduction de l'oxyde de triméthylamine OTMA en triméthylamine TMA peut être détectée à l'aide de capteurs appelés « nez électroniques » ou de biocapteurs.

Le plus souvent, ces nez électroniques comportent des capteurs en oxyde métallique semi conducteurs. Un exemple de nez électronique (Gemini, société Alpha MOS, Toulouse) pour l'évaluation de la qualité du saumon est décrit dans Haugen et al, Sensors and actuators B 116, pp. 72-77, 2006. On connaît également des nez électroniques à polymères (LibraNose ou EnQbe, société Tor Vergata, voir Macagnano et al, A model to predict fish quality from instrumental features, Sensors and actuators B 111-112, pp. 293-298, 2005). On connaît aussi des nez électroniques à quartz, des nez électroniques à capteurs électrochimiques (FreshSense, société Element Bodvaki, voir Natale et al, Comparison and integration of different electronic noses for freshness evaluation of cod-fish fillets, Sensors and actuators B 77, pp. 572-578, 2001).

Les nez électroniques permettent l'identification des composés volatiles produits lors de la détérioration du poisson. Des profils olfactifs sont établis pour chaque espèce considérée.

Les nez électroniques sont coûteux.

L'environnement de la criée (hygrométrie, température basse, circulation des personnes et des engins, produits désinfectants) et la forte variabilité des produits de la mer (grande variété d'espèces, modification des caractéristiques par la saison, la nutrition, le type de pêche, la saignée, le glaçage) rend complexe l'analyse de la qualité du poisson par nez électronique.

Méthodes mettant en oeuvre des mesures de paramètres physiques

Les méthodes mettant en oeuvre des mesures de paramètres physiques sont également très variées :

- mesures de la couleur (Schubring, Colour measurement for the determination of freshness of fish, Quality of fish from catch to the consumer, pp. 251-263, 2003),
- analyse d'images permettant d'évaluer la brillance de la peau, la turbidité du gel qui couvre la peau ou sa couleur ; la convexité de l'oeil, la noirceur de la rétine et sa transparence, « gaping » (mesure de la dépression) de la chair,
- propriétés électriques : changement des propriétés électriques du muscle du poisson après la mort (voir par exemple Oehlenschlager, Measurement of freshness quality of fish based on electrical properties, Quality of fish from catch to consumer labelling, monitoring and tracebility, pp. 237-249, 2003) ;
- spectroscopie, par exemple spectroscopie de fluorescence frontale, permettant une mesure en ligne : voir par exemple le document WO 2003/048761).

Yapar et al (Determination of anchovy freshness by refractive index or eye fluid, Food Research International, Vol. 31, N° 10, pp. 693-695, 1998), présentent les résultats de mesure d'indice de réfraction de l'oeil et de mesures de concentration en TMA pour l'espèce *Engraulis encrasicolus*, et concluent à une bonne corrélation entre les deux techniques d'évaluation de la fraîcheur de ce poisson. Des résultats analogues ont été obtenus par Gokoglu et al, pour l'espèce Sardina pilchardus (Use of eye fluid refractive index in sardine as a freshness indicator, European Food Research and Technology, Vol. 218, N° 3, pp. 295-297, 2004). La mesure de l'indice de réfraction de l'oeil nécessite d'extraire l'oeil du poisson, de sorte que cette méthode est destructive et longue.

L'analyse d'image et la vision par ordinateur ont été proposées pour l'inspection de poissons (Brosnan et al, Improving quality inspection of food products by computer vision- a review, Journal of food engineering 61, pp. 3-16, 2004). Les premières applications concernaient le tri en ligne de poissons, par espèces, par sexe ou par calibre (voir par exemple Zion et al ; Sorting fish by computer vision, Computers and eclectronics in agriculture 23, pp. 175-187, 1999). La vision par ordinateur a également été employée, au stade expérimental, pour la détection d'arêtes dans les filets de poissons (Pau et al, Fish quality control by computer vision, ISBN 082478426X). Mais pas pour la détermination de la fraîcheur du poisson.

Combinaisons de techniques

La possibilité de développer un dispositif multi capteurs pour la détermination de la fraîcheur du poisson a fait l'objet du programme FAIR CT 98-4076 (Mustec). Pour ce programme européen, on peut se reporter par exemple aux documents suivants : Olafsdottir at al Multisensor for fish quality determination, Trends in food science & technology 15, pp.86-93, 2004 ; www.mustec.difres.dk. Toutes les techniques utilisées dans le projet Mustec étaient non destructives : nez électroniques, analyse d'images, QIM, spectroscopie, propriétés électriques du muscle du poisson.

Associant un nez électronique (provenant de la société AlphaMos) et une analyse d'images, le projet *eFish*, en cours de développement depuis 1999 propose un dispositif de contrôle de la fraîcheur du poisson, les données issues de ce dispositif étant analysées à distance. Le document FR 2829578 décrit le procédé eFish. Ainsi qu'il est indiqué dans ce document, après une phase d'apprentissage, les gammes de valeurs discriminantes des analyses optique, chimique, et des données de contexte sont établies. L'analyse optique met en oeuvre une reconnaissance de formes et de couleurs. Un modèle est construit selon une méthode supervisée (connaissance a priori d'une loi de répartition) ou une méthode non supervisée (algorithme des centres mobiles). Des données de contexte sont prises en compte : type de produit (espèce du poisson), type d'unité de produit (individuel ou en vrac), date de prise du produit, conditions de prise du produit (chalut, filet, ligne, élevage, profondeur de pèche, lieu de pèche), conditions de transport et de stockage du produit, date du contrôle. Au moins une partie des données de contexte est enregistrée par lecture automatique d'une étiquette passive ou active à lecture optique ou audio.

Problèmes techniques à résoudre

Pour l'évaluation de la fraîcheur du poisson, une des premières questions qui se posent est de définir précisément la notion de « fraîcheur », en associant cette définition à des paramètres ou indicateurs.

La fraîcheur du poisson doit avoir un lien avec les propriétés organoleptiques du produit et avec ses propriétés microbiologiques, garantes de la sécurité alimentaire.

Il n'existe pas aujourd'hui de paramètre ou indicateur de fraîcheur qui soit tout à la fois simple, objectif, déterminé par une technique de mesure fiable, reproductible, peu coûteuse, rapide à mettre en oeuvre et indépendante de l'opérateur comme de l'environnement de la mesure.

Un tel paramètre ou indicateur serait d'un grand intérêt. Reconnu par tous les intervenants de la filière comme un indice de qualité et de valeur, un tel indicateur ou paramètre permettrait à la notion de fraîcheur du poisson d'intervenir plus directement qu'aujourd'hui dans les signes de qualité (par exemple indication géographique protégée, label rouge, appellation d'origine contrôlée, certificats de conformité).

La plupart des méthodes d'évaluation de fraîcheur de l'état de la technique ne sont pas utilisées pour mesurer la perte de fraîcheur dans les heures qui suivent la mort du poisson.

La plupart des méthodes d'évaluation de fraîcheur de l'état de la technique ne permettent pas l'estimation en continu, à la volée, en particulier lors d'une vente électronique aux enchères à la criée.

La plupart des méthodes d'évaluation de fraîcheur de l'état de la technique ne permettent pas non plus une estimation rapide de cette fraîcheur avant l'arrivée du poisson à la criée, ce qui ne facilite pas la gestion de l'information pour la prévente et l'achat à distance.

Il existe un besoin d'une définition de la fraîcheur du poisson par des paramètres ou indicateurs objectifs, permettant la mesure de cette fraîcheur par une technique peu onéreuse, indépendante de l'opérateur et de l'environnement de l'appareil de mesure.

Plus précisément, il existe un besoin d'une méthode de détermination de la fraîcheur du poisson qui soit tout à la fois :

- associable à une ou plusieurs caractéristiques sensorielles,
- tenant compte du temps passé après capture, par exemple lors du stockage sous glace ;
- de coût peu élevé,
- sensible aux premiers signes d'altération du poisson,
- indépendante de variables a priori extrinsèques aux qualités gustatives, telles que le poids ou la taille du poisson,

cette méthode permettant une plus grande fiabilité des achats en criée et à distance et fournissant des résultats qui puissent être comparées aux autres méthodes utilisées actuellement.

L'invention vise à fournir un procédé et un dispositif permettant de résoudre les problèmes mentionnés ci-dessus.

A cette fin, l'invention se rapporte, selon un premier aspect, à un procédé de contrôle non destructif de la qualité du poisson, ce procédé comprenant une mesure biométrique du poisson, ce procédé comprenant une étape de détection de l'oeil du poisson par un algorithme de détection d'arcs circulaires basés sur les contours, un cumul des centroïdes supposés pouvoir être le centre d'une pupille étant ensuite effectué, les zones de cumul important étant extraites, l'adresse de leur centroïdes étant moyennée pour obtenir le centre le plus probable.

Avantageusement, le procédé comprend une étape de pré-calcul de tableaux d'adresses de centroïdes connaissant trois points du contour.

Dans une mise en oeuvre, les contours de la pupille et du blanc de l'oeil étant supposés circulaires, N histogrammes angulaires Hi sont calculés, chaque histogramme correspondant à un rayon, pour chaque centre le plus probable de l'oeil. Selon diverses réalisations, le procédé comprend les caractères suivants, le cas échéant combinés :

- le nombre d'histogrammes N vaut 360, i variant de 0° à 360°, les rayons étant équidistants.
- une valeur C(R) est calculée, pour chaque rayon R, selon la formule suivante :

$$C(R)= \text{somme des Hi} \times \text{Maximum des Hi}$$

la première transition pupille vers blanc (PB) de l'oeil correspondant à la grande pente ascendante de la valeur C(R), la seconde transition entre blanc de l'oeil et extérieur de l'oeil (BE) correspondant au plus fort minimum qui suit le maximum du blanc pupillaire.

- une analyse d'images est effectuée sur des petites images à résolution faible et fixe, par exemple 640x480 pixels, ces petites images étant prises à l'intérieur d'une image haute résolution par exemple 2000x1500 pixels.
- plus de douze images 640x480 pixels sont prises dans une image 2000x1500 pixels, et plus particulièrement une vingtaine d'images 640x480 pixels.
- les petites images sont échantillonnées par combinaison mixte de sélection aléatoire et de sélection selon une fonction de probabilité de position de poisson.
- les petites images sont échantillonnées de manière aléatoire, les adresses horizontales et verticales de l'image à analyser étant aléatoires.L'invention se rapporte selon un deuxième aspect, à un dispositif pour la mise en oeuvre du procédé présenté ci-dessus, le dispositif comprenant des moyens d'analyse des images capturées, et des moyens d'éclairage indirect et diffusant de la lumière produite par des moyens d'éclairage.

Avantageusement, au moins deux caméras sont disposées au dessus du bac de criée, les champs de vision de ces caméras se recouvrant partiellement.

Selon diverses réalisations, le dispositif présente les caractères suivants, le cas échéant combinés :

- les moyens d'éclairage diffusant et indirect comprennent une plaque, placée au dessus des poissons, et notamment au dessus d'un bac de criée, cette plaque formant un tunnel.
- la plaque est d'une longueur plus importante que celle du bac de criée contenant les poissons.
- les moyens d'éclairage sont disposés de part et d'autre du bac de criée.
- la plaque formant tunnel est cintrée, moulée ou injectée ou en inox.
- la plaque est de qualité reconnue pour son usage en environnement alimentaire, par exemple en polystyrène.

D'autres objets et avantages de l'invention apparaîtront au cours de la description suivante de modes de réalisation actuellement préférés, description qui va être effectuée en se référant aux dessins annexés dans lesquels :

- la figure 1 est une vue schématique d'un dispositif d'éclairage diffusant d'un bac de criée ;
- la figure 2 est un schéma montrant le principe d'obtention d'histogrammes angulaires, à partir d'un centre hypothétique de pupille d'oeil de poisson ;
- la figure 3 est une courbe C(R) théorique ;
- la figure 4 est une courbe C(R) pour un oeil de dorade grise ;
- la figure 5 est une vue de dessus de dorade grises, sur lesquelles sont reportées les axes de mesure de la distance museau/oeil ;
- la figure 6 est une vue d'une courbe C(R) pour un oeil de bar, quatre jours après la mort ;
- la figure 7 est une vue d'une courbe C(R) pour un oeil de bar, sept jours après sa mort.

Le procédé selon l'invention met en oeuvre des mesures biométriques de l'oeil des poissons.

L'oeil des poissons, comme pour tous les vertébrés, comprend, en arrière de la cornée, une pupille (ouverture dans l'iris) au travers de laquelle la lumière parvient à la rétine, après avoir traversé le cristallin. Le cristallin de l'oeil des poissons est pratiquement sphérique et n'est pas déformable. Un muscle lenticulaire permet le déplacement du cristallin. La plus grande partie de la surface du globe oculaire est constitué de tissu fibreux dur et flexible, la sclérotique (blanc de l'oeil). Derrière le cristallin se trouve un corps gélatineux, le vitré. La forme de l'oeil est maintenue par la sécrétion de l'humeur aqueuse.

L'aspect de l'oeil du poisson est utilisé de manière empirique par les consommateurs ou les négociants pour estimer la fraîcheur du poisson. En effet, une perte en eau et une augmentation de la perméabilité membranaire impliquent une réduction de la pression intraoculaire modifiant la transmission de la lumière. L'altération du fluide oculaire peut également affecter les propriétés de réfraction de la lumière.

Il s'avère que l'oeil est la partie du poisson la plus délicate à détecter en vision par ordinateur ou analyse d'images, et ce pour deux raisons principales.

Premièrement, l'oeil des poissons est de petite taille. C'est en valeur absolue le cas par exemple pour les soles ou les lamproies. D'une manière générale, pour les poissons destinés à la consommation humaine, l'oeil est d'une taille relative très petite par rapport au corps de l'animal.

Deuxièmement, l'oeil des poissons est généralement circulaire. Or, d'une manière générale, en traitement d'images, il est plus difficile de détecter des éléments de contour circulaires que des éléments de contours rectilignes parce que la détection d'éléments circulaires requiert un temps de calcul beaucoup plus important. Le temps de calcul devient critique pour des images de plusieurs millions de pixels. Pour que le temps de calcul avoisine quelques secondes au plus, il est fondamental que les algorithmes de détection spécifiques de contour circulaire soient rapides.

La détection automatique et rapide de l'oeil dans le corps de poissons disposés en vrac dans un bac de criée est donc une opération en elle-même complexe. Il est à noter que le procédé selon l'invention met en oeuvre des mesures biométriques de l'oeil des poissons.

Il s'avère que la détection de l'oeil et de la pupille des poissons est très délicate en vision par ordinateur ou analyse d'images, notamment par suite de l'assombrissement du blanc de l'oeil ou par suite d'un mauvais contraste d'images, la pupille étant censée être sombre et contrastée.

Le procédé selon l'invention permet la mesure de paramètres biométriques de poissons disposés en vrac dans des bacs de criées.

Un bac de criée est conventionnellement un bac à fond rectangulaire, emboîtable, gerbable, pourvu le plus souvent de moyens de drainage. Fabriqué par exemple en PEHD, sa hauteur est par exemple de 100 à 270 mm.

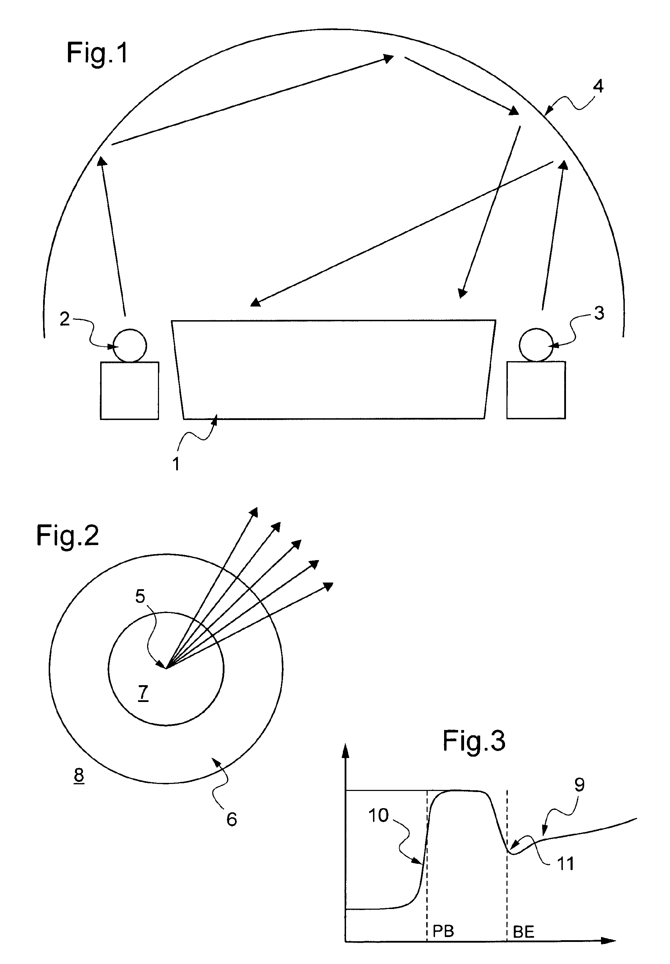

Ainsi qu'il apparaît en figure 1, le bac de criée 1 est éclairé de manière indirecte et diffusante, la lumière produite par des moyens d'éclairage 2, 3 subissant des réflexions successives sur une plaque 4, placée au dessus du bac de criée 1, cette plaque 4 formant un tunnel. La plaque 4 est d'une longueur plus importante que celle du bac de criée 1, de sorte qu'aucune source de lumière extérieure n'éclaire le bac à ses extrémités.

L'éclairage diffusant limite le risque de formation d'ombres et de reflets.
Dans le mode de réalisation représenté, les moyens d'éclairage 2, 3 sont disposés latéralement, de part et d'autre du bac de criée 1. Dans un mode de réalisation, ces moyens d'éclairage sont des éclairages pour utilisation avec des produits alimentaires. La plaque 4 formant tunnel au dessus du bac peut être une plaque cintrée, moulée ou injectée ou en inox. Dans un mode de mise en oeuvre, cette plaque est en polystyrène.

L'éclairage du bac de criée 1 est ainsi avantageusement indépendant de toute source d'éclairage extérieure et le bac de criée 1 est uniformément éclairé.

La demanderesse a en effet constaté qu'une variation d'éclairage peut engendrer une dérive des mesures portant sur des petits objets tels que des pupilles d'oeil de poisson. C'est en particulier le cas lorsque les poissons sont empilés en vrac dans le bac de criée, et que la tête de certains poissons se trouve au voisinage d'un des quatre coins du bac de criée.

Les variations d'éclairage pourraient par ailleurs augmenter la probabilité d'artéfacts.

Le dispositif permet ainsi de s'affranchir des différences d'éclairage des criées.

Le bac de criée 1 contient des poissons. Ces poissons peuvent être, et sont le plus souvent, disposés en vrac. Ces poissons peuvent être, et sont le plus souvent, empilés en vrac.

Des moyens de capture d'image sont disposés au dessus du bac de criée. Ces moyens peuvent prendre plusieurs formes : appareil photographique numérique, caméra numérique, caméra CCD (*Charged Couple Device*).

Dans un mode de réalisation, deux caméras sont disposées au dessus du bac de criée 1, les champs de vision de ces caméras se recouvrant partiellement.

Cette disposition est avantageuse lorsque le bac de criée a un fond rectangulaire, compte tenu du coût des caméras industrielles de haute résolution : une caméra de résolution 3000x2000 pixels environ est beaucoup plus onéreuse que deux caméras de résolution 2000x1500 pixels.

Dans une mise en oeuvre, les caméras sont à haute résolution (2000x1500 pixels) et fonctionnent en mode photo.

Dans une autre mise en oeuvre, les caméras sont à basse résolution (640x480 pixels) et fonctionnent en mode vidéo (par exemple caméscope, webcam, caméra industrielle Firexware® ou USB).

Avantageusement, la caméra ou l'appareil de photographie numérique est placée à une distance telle que la pupille a un rayon de 25 à 30 pixels. Avantageusement, l'image obtenue par la caméra ou l'appareil photographique est nette pour des poissons disposés sensiblement à mi profondeur du bac de criée, et la profondeur de champ est adaptée à la profondeur du bac de criée. A titre indicatif, la distance de mise au point est de l'ordre de 60 cm, lorsque la prise de vue est effectuée en mode photo, et de 15 à 20 cm lorsque la prise de vue est effectuée en mode vidéo.

Avantageusement, la résolution de chacune des deux caméras est de 2000x1500 pixels.

Dans une première étape du procédé de vision par ordinateur, une analyse d'images est effectuée sur des petites images à résolution faible et fixe, par exemple 640x480 pixels, ces petites images étant prises à l'intérieur d'une image haute résolution (par exemple 2000x1500 pixels).

Avantageusement, plus de douze images 640x480 pixels sont prises dans une image 2000x1500 pixels. De sorte à éviter les effets de bord, une vingtaine d'images 640x480 pixels sont à analyser pour chacune des deux caméras.

Cette analyse d'images à résolution faible et fixe présente de nombreux avantages. En premier lieu, l'échantillonnage aléatoire des petites images permet l'obtention des premiers résultats de l'analyse biométrique, sans attendre que toutes les petites images soient échantillonnées.

Dans un mode de réalisation, l'échantillonnage est effectué par sélection non linéaire, par combinaison mixte de sélection aléatoire et de sélection selon une fonction de probabilité de position de poisson. Ce mode de réalisation est avantageux lorsque les têtes de poisson sont le plus souvent positionnées aux extrémités du bac de criée.

Dans un autre mode de réalisation, l'échantillonnage est aléatoire, les adresses horizontales et verticales de l'image à analyser étant aléatoires.

La détection de l'oeil met en oeuvre un algorithme de détection d'arcs circulaires basés sur les contours. Puis, l'on effectue un cumul des centroïdes supposés pouvoir être le centre d'une pupille. Enfin, les zones de cumul important sont extraites et l'adresse de leur centroïdes est moyennée pour obtenir le centre le plus probable, à une précision sub-pixellique.

Des tableaux pré-calculés d'adresses de centroïdes connaissant trois points du contour sont utilisés pour réduire le temps de calcul.

Pour l'analyse biométrique de l'oeil, les contours de la pupille et du blanc de l'oeil sont supposés circulaires.

Pour chaque centre d'oeil hypothétique, c'est-à-dire pour chaque centre le plus probable 5, N histogrammes angulaires Hi sont calculés, i variant de 0° à 360°, ainsi qu'il est représenté schématiquement en figure 2. Le nombre d'histogrammes dépend du nombre de pixels balayés par la circonférence du blanc 6. Par exemple N vaut 360.

Un histogramme théorique est représenté en figure 3.

On calcule la valeur C(R), selon la formule suivante, pour chaque rayon R :

$$C(R)= \text{somme des Hi x Maximum des Hi}$$

Cette formule permet de tenir compte des deux cas de figure extrême suivants :

- lorsque les contrastes entre la pupille 7 et le blanc 6 d'une part et entre le blanc 6 et l'extérieur 8 de l'oeil d'autre part sont présents pour tous les histogrammes 9, les histogrammes sont avantageusement cumulés ;
- lorsque les contrastes sont présents pour certains angles mais pas pour d'autres, c'est la valeur maximale de niveau de gris qui est prise en compte, pour tous les angles, à rayon donné.

La demanderesse a constaté que la transition entre la pupille 7 et le blanc 6 est marquée, dans le cas de la dorade, par un fort contraste entre le noir pupillaire et le niveau de gris du blanc de l'oeil.

La demanderesse a constaté que la transition entre le blanc 6 de l'oeil et l'extérieur 8 de l'oeil n'est pas, dans le cas de la dorade, aussi marquée que la transition entre la pupille 7 et le blanc 6 de l'oeil, le niveau de gris de l'extérieur de l'oeil pouvant être presque aussi clair que le blanc.

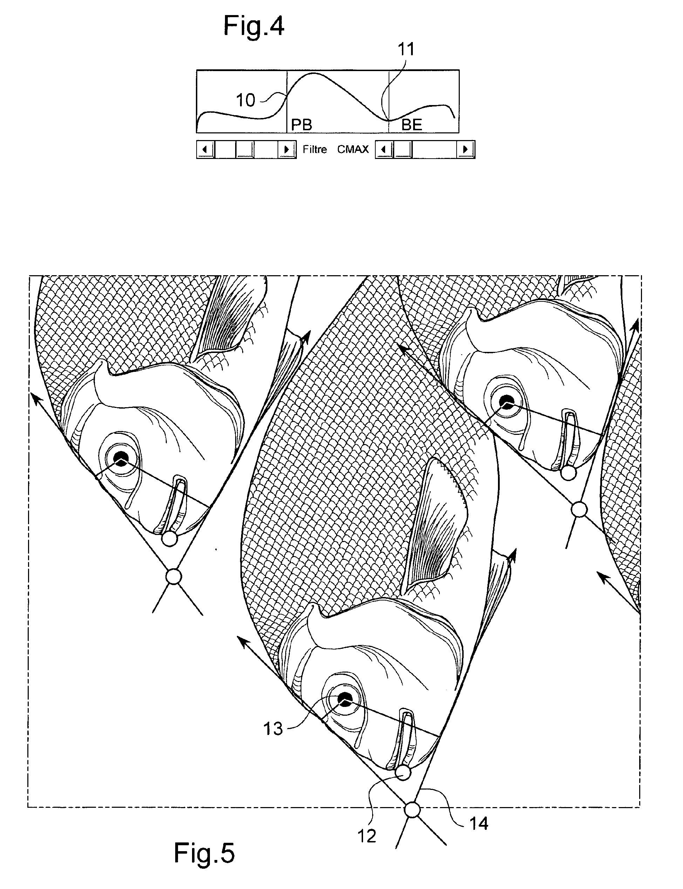

La figure 4 montre un exemple de courbe C(R) obtenu pour une dorade grise (*Sparus aurata*). La première transition pupille vers blanc (PB) de l'oeil correspond à la grande pente ascendante 10. La seconde transition entre blanc de l'oeil et extérieur de l'oeil (BE) correspond au plus fort minimum 11 qui suit le maximum du blanc pupillaire, ce minimum étant lié à la courbure de l'oeil.

Les contours externes du poisson sont extraits, pour permettre la mesure de distance et d'angles, par exemple la distance entre le bout du museau 12 et l'oeil 13.

Cette mesure est effectuée de la manière suivante.

Dans un premier temps, le dispositif de vision par ordinateur (par la suite le dispositif) recherche parmi l'ensemble des points de contours externe celui qui est le plus proche du centre de la pupille.

Dans un deuxième temps, le dispositif retient l'ensemble des points de contour autour de ce point qui sont à une distance de l'oeil comprise entre la distance minimale et une distance multiple (par exemple double).

Dans un troisième temps, le dispositif calcule la direction prépondérante du segment de contour ainsi calculé.

Dans un quatrième temps, le dispositif calcule l'équation de la droite qui possède cette direction prépondérante et qui est tangent au segment de contour calculé.

Dans un cinquième temps, le dispositif recherche parmi l'ensemble des points de contour externe celui dont le centre de courbure est le plus proche du centre de la pupille.

Dans un sixième temps, le dispositif retient l'ensemble des points de contour externe dont la distance du centre de courbure à l'oeil est située entre ce minimum et un maximum.

Puis, le dispositif calcule la direction prépondérante de ce deuxième ensemble de points et calcule l'équation de la droite tangente.

L'on obtient ainsi le repère 14 représenté en figure 5.

La localisation de l'oeil est directe et permet ensuite le calcul du repère 14 représentant une partie de la géométrie du poisson.

Une caractérisation biométrique du poisson, à l'intérieur de cette géométrie, peut inclure la mesure de la brillance ou niveau de gris moyen, la mesure de la couleur ou de la coloration moyenne, la mesure du contraste de texture (motifs texturés comme par exemple les écailles).

L'extraction de contours est avantageusement réalisée en utilisant des librairies informatiques d'extraction de contours qui sont disponibles auprès de la société URATEK sous le label URACODE 2008 et URACER.

L'on décrit maintenant les résultats obtenus pour l'espèce *Dicentarchus labrax* (bar).

L'oeil du bar présente des différences par rapport à celui de la dorade : la pupille est moins ronde et la sclérotique est plus foncée, la membrane recouvrant l'oeil est plus opaque.

Six spécimens, issus d'élevage, ont été stockés dans un réfrigérateur de 76 litres, la température variant entre 3,8°C et 5,6°C.

Le bas de criée 1 est rectangulaire (60cmx80cm).

L'appareil photo numérique avait une résolution de 6 millions de pixels.

Un ordinateur portable avec processeur Intel Core 2 T 5200 (1,6 GHz) et 1 Go de mémoire vive est employé pour le programme d'analyse d'images.

Les moyens d'éclairage 3 sont deux tubes fluorescents de 18W chacun et 58 cm de longueur.

La plaque 4 cintrée est en polystyrène de 0,4 cm d'épaisseur et mesure 1 m x 1,25 m. Elle est placée de sorte à former un tunnel de 45 cm de hauteur.

Chaque spécimen a été pesé et mesuré avant le début des mesures biométriques.

|                | Poisson 1 | Poisson 2 | Poisson 3 | Poisson 4 | Poisson 5 | Poisson 6 |
|----------------|-----------|-----------|-----------|-----------|-----------|-----------|
| Poids (g)      | 392       | 378       | 394       | 321       | 315       | 430       |
| Longueur (mm)  | 320       | 325       | 325       | 300       | 305       | 330       |

Des séries de six photographies ont été prises à chaque intervalle de temps. Pour les trois premières photos, le poisson est placé parallèlement à la longueur du bac. Pour les trois dernières photos, le poisson est placé perpendiculairement à la longueur du bac de criée 4.

Après le troisième jour, trois photos réunissant l'ensemble des spécimens sont prises à chaque intervalle de temps, en plus de photos individuelles de chaque spécimen.

Une moyenne de cinq séries de photos par jour sont prises, à raison de deux à cinq heures d'intervalle pour le premier jour et de cinq heures d'intervalle de temps pour les jours suivants.

Une base de données photographique du bar est constituée, dès les premières heures après la mort, estimée à quatre heures avant la première prise de vue, et ce jusqu'au septième jour après la mort.

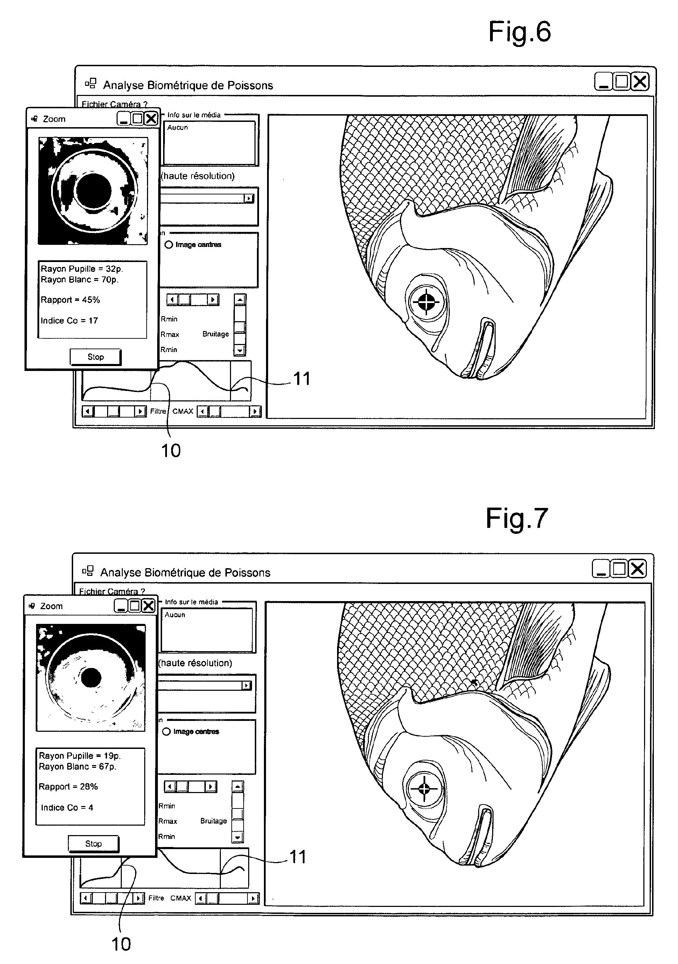

Au cours du temps, l'oeil rond et globuleux devient effacé, puis ellipsoïdal. La mesure du paramètre C(R) et l'identification des transitions 10, 11 pupille/blanc et blanc/extérieur de l'oeil de quantifier cette transformation, ainsi qu'il est visible en figures 6 et 7.

En mettant en correspondance les valeurs biométriques de l'oeil et les résultats d'autres méthodes d'évaluation de la qualité du poisson, présentées en introduction, il est possible d'étalonner le procédé selon l'invention.

Le procédé selon l'invention met avantageusement en oeuvre la détection et la caractérisation de l'oeil du poisson et ce alors même que l'oeil est difficile à détecter du fait de sa petite taille et des artéfacts perturbateurs (notamment mauvais éclairement de la tête du poisson, placée dans un angle du bac). L'éclairage diffusant du bac, indépendant de toute source d'éclairage extérieur permet de réduire les artéfacts. L'utilisation d'algorithme de détection d'arcs circulaires basés sur les contours et l'utilisation de librairies informatiques d'extraction de contours permettent des mesures biométriques oculaires sur des yeux de poissons dont la pupille ne représente qu'un millième de la surface de l'image.

**Revendications**

1.  Procédé de contrôle non destructif de la qualité du poisson, ce procédé comprenant une mesure biométrique du poisson, **caractérisé en ce qu'**il comprend une étape de détection de l'oeil du poisson par un algorithme de détection d'arcs circulaires basés sur les contours, un cumul des centroïdes supposés pouvoir être le centre d'une pupille étant ensuite effectué, les zones de cumul important étant extraites, l'adresse de leur centroïdes étant moyennée pour obtenir le centre le plus probable.

2.  Procédé selon la revendication 1, **caractérisé en ce qu'**il comprend une étape de pré-calcul de tableaux d'adresses de centroïdes connaissant trois points du contour.

3.  Procédé selon la revendication 1 ou 2, **caractérisé en ce que**, les contours de la pupille et du blanc de l'oeil étant supposés circulaires, N histogrammes angulaires Hi sont calculés, chaque histogramme correspondant à un rayon, pour chaque centre le plus probable (5) de l'oeil.

4.  Procédé selon la revendication 3, **caractérisé en ce que** N vaut 360, i variant de 0° à 360°, les rayons étant équidistants.

5.  Procédé selon la revendication 3 ou 4, **caractérisé en ce que** une valeur C(R) est calculée, pour chaque rayon R, selon la formule suivante :

$$C\,(R) = \text{somme des Hi x Maximum des Hi}$$

la première transition pupille vers blanc (PB) de l'oeil correspondant à la grande pente ascendante (10) de la valeur C(R), la seconde transition entre blanc de l'oeil et extérieur de l'oeil (BE) correspondant au plus fort minimum (11) qui suit le maximum du blanc pupillaire.

6.  Procédé selon l'une quelconque des revendications précédentes, **caractérisé en ce qu'**une analyse d'images est effectuée sur des petites images à résolution faible et fixe, par exemple 640x480 pixels, ces petites images étant prises à l'intérieur d'une image haute résolution par exemple 2000x1500 pixels.

7.  Procédé selon la revendication 6, **caractérisé en ce que** plus de douze images 640x480 pixels sont prises dans une image 2000x1500 pixels, et plus particulièrement une vingtaine d'images 640x480 pixels.

8.  Procédé selon la revendication 6 ou 7, **caractérisé en ce que** les petites images sont échantillonnées par combinaison mixte de sélection aléatoire et de sélection selon une fonction de probabilité de position de poisson.

9.  Procédé selon la revendication 6 ou 7, **caractérisé en ce que** les petites images sont échantillonnées de manière aléatoire, les adresses horizontales et verticales de l'image à analyser étant aléatoires.

**10.** Dispositif pour la mise en oeuvre du procédé selon l'une quelconque des revendications précédentes, **caractérisé en ce qu'**il comprend des moyens de capture d'image, des moyens d'analyse des images capturées, et des moyens d'éclairage indirect et diffusant de la lumière produite par des moyens d'éclairage (2, 3).

**11.** Dispositif selon la revendication 10, **caractérisé en ce qu'**au moins deux caméras sont disposées au dessus du bac de criée (1), les champs de vision de ces caméras se recouvrant partiellement.

**12.** Dispositif selon la revendication 10 ou 11, **caractérisé en ce que** les moyens d'éclairage diffusant et indirect comprennent une plaque (4), placée au dessus des poissons, et notamment au dessus d'un bac de criée (1), cette plaque (4) formant un tunnel.

**13.** Dispositif selon la revendication 12, **caractérisé en ce que** la plaque (4) est d'une longueur plus importante que celle du bac de criée (1) contenant les poissons.

**14.** Dispositif selon l'une quelconque des revendications 10 à 13, **caractérisé en ce que** les moyens d'éclairage (2, 3) sont disposés de part et d'autre du bac de criée (1).

**15.** Dispositif selon l'une quelconque des revendications 10 à 14, **caractérisé en ce que** les moyens d'éclairage sont des éclairages pour utilisation avec des produits alimentaires.

**16.** Dispositif selon l'une quelconque des revendications 10 à 15, **caractérisé en ce que** la plaque (4) formant tunnel est cintrée, moulée ou injectée ou en inox.

**17.** Dispositif selon la revendication 16, **caractérisé en ce que** cette plaque est de qualité reconnue pour son usage en environnement alimentaire.

Fig.1

2

3

4

1

Fig.2

5

7

8

6

Fig.3

10

9

11

PB          BE

## Fig.4

## Fig.5

## Fig.6

## Fig.7

Europäisches
Patentamt

European
Patent Office

Office européen
des brevets

**RAPPORT DE RECHERCHE EUROPEENNE**

Numéro de la demande

EP 09 29 0878

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | WO 2008/016309 A (SINVENT AS [NO]; JANSSON STIG [NO]; MATHIASSEN JOHN REIDAR [NO]) 7 février 2008 (2008-02-07) * page 2, ligne 24 - page 3, ligne 21 * * page 3, ligne 32 - page 4, ligne 2; figures 1,2 * | 10-17 | INV. G01N33/12 |
| X | GB 2 446 822 A (ENFIS LTD [GB]) 27 août 2008 (2008-08-27) * page 1, ligne 14-16 * * page 8, ligne 28 - page 9, ligne 16; figure 1 * | 10-17 | |
| A | WO 2008/017104 A (BUHI INTERNAT GROUP BIG PTY LT [AU]; COMANDANTE BONIFACIO F [AU]) 14 février 2008 (2008-02-14) * page 9, ligne 21 - page 10, ligne 8; tableau 1 * | 1,10 | |
| A | US 2003/072472 A1 (HAAGENSEN PETER [US] ET AL HAAGENSEN PETER [US] ET AL) 17 avril 2003 (2003-04-17) * alinéas [0025], [0033] - [0070]; figures 1,4,5 * | 1-10 | DOMAINES TECHNIQUES RECHERCHES (IPC)  G01N A22C |
| A | US 4 631 413 A (JENSEN SVEND A K [DK] ET AL) 23 décembre 1986 (1986-12-23) * colonne 3, ligne 22-30 * * colonne 5, ligne 1-24; figure 9 * | 1,10 | |
| A | RU 2 180 112 C2 (KALININGR G TEKHN UNIV; ERSITET) 27 février 2002 (2002-02-27) * abrégé * | 1,10 | |

Le présent rapport a été établi pour toutes les revendications

| Lieu de la recherche | Date d'achèvement de la recherche | Examinateur |
|---|---|---|
| La Haye | 17 février 2010 | Wulveryck, J |

CATEGORIE DES DOCUMENTS CITES

X : particulièrement pertinent à lui seul
Y : particulièrement pertinent en combinaison avec un
    autre document de la même catégorie
A : arrière-plan technologique
O : divulgation non-écrite
P : document intercalaire

T : théorie ou principe à la base de l'invention
E : document de brevet antérieur, mais publié à la
    date de dépôt ou après cette date
D : cité dans la demande
L : cité pour d'autres raisons
.................................................................
& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 09 29 0878

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

17-02-2010

| Document brevet cité au rapport de recherche | | | Date de publication | Membre(s) de la famille de brevet(s) | | Date de publication |
|---|---|---|---|---|---|---|
| WO 2008016309 | A | | 07-02-2008 | AUCUN | | |
| GB 2446822 | A | | 27-08-2008 | WO | 2008102143 A1 | 28-08-2008 |
| WO 2008017104 | A | | 14-02-2008 | AUCUN | | |
| US 2003072472 | A1 | | 17-04-2003 | CA | 2367640 A1 | 15-04-2003 |
| | | | | CA | 2642046 A1 | 15-04-2003 |
| | | | | EP | 1449154 A1 | 25-08-2004 |
| | | | | NO | 20040602 A | 04-05-2004 |
| | | | | WO | 03034319 A1 | 24-04-2003 |
| | | | | US | 2004125987 A1 | 01-07-2004 |
| US 4631413 | A | | 23-12-1986 | DE | 3473797 D1 | 06-10-1988 |
| | | | | DK | 287584 A | 14-12-1984 |
| | | | | EP | 0128889 A1 | 19-12-1984 |
| | | | | NO | 842344 A | 14-12-1984 |
| RU 2180112 | C2 | | 27-02-2002 | AUCUN | | |

Pour tout renseignement concernant cette annexe : voir Journal Officiel de l'Office européen des brevets, No.12/82

EPO FORM P0460

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 240696 A
- WO 2008056990 A
- EP 137677 A

- WO 2003048761 A
- FR 2829578

**Littérature non-brevet citée dans la description**

- **Nicolle et al.** *Les conserves des produits de la mer,* ISSN 0998-4089, www.ifremer.fr
- **Sykes et al.** Assessment of European cuttlefish nutritional value and freshness under ice storage using a developed QIM and biochemical methods. *LUVT Food Science and Technology,* 2008
- **Bonilla et al.** Development of QIM scheme for fresh cod fillets and application in shelf life study. *Food Control,* 2007, vol. 18, 352-358
- **Pires et al.** Development of new QIM schemes for cuttlefish and broadtail short fin squid. *Food control,* 2006, vol. 17, 942-949
- **Okuma et al.** Flow system for fish freshness determination based on double multienzyme reactor electrodes. *Biosensors & bioelectronics,* 2002, vol. 17, 367-372
- **Haugen et al.** *Sensors and actuators B,* 2006, vol. 116, 72-77
- **Macagnano et al.** A model to predict fish quality from instrumental features. *Sensors and actuators B 111-112,* 2005, 293-298
- **Natale et al.** Comparison and integration of different electronic noses for freshness evaluation of cod-fish fillets. *Sensors and actuators B,* 2001, vol. 77, 572-578

- **Schubring, Colour measurement for the determination of freshness of fish.** *Quality of fish from catch to the consumer,* 2003, 251-263
- **Oehlenschlager, Measurement of freshness quality of fish based on electrical properties.** *Quality of fish from catch to consumer labelling, monitoring and tracebility,* 2003, 237-249
- **Yapar et al.** Determination of anchovy freshness by refractive index or eye fluid. *Food Research International,* 1998, vol. 31 (10), 693-695
- **Gokoglu et al.** Use of eye fluid refractive index in sardine as a freshness indicator. *European Food Research and Technology,* 2004, vol. 218 (3), 295-297
- **Brosnan et al.** Improving quality inspection of food products by computer vision- a review. *Journal of food engineering,* 2004, vol. 61, 3-16
- **Zion et al.** *Sorting fish by computer vision, Computers and eclectronics in agriculture,* 1999, vol. 23, 175-187
- **Pau et al.** *Fish quality control by computer vision,* ISBN 082478426X
- **Olafsdottir.** Multisensor for fish quality determination. *Trends in food science & technology,* 2004, vol. 15, 86-93, www.mustec.difres.dk